Europäisches Patentamt

European Patent Office

Office européen des brevets

(11) Veröffentlichungsnummer: **0 425 714 A1**

# EUROPÄISCHE PATENTANMELDUNG

(21) Anmeldenummer: 89120072.7

(22) Anmeldetag: 28.10.89

(51) Int. Cl.5: **A61F 2/30**

(43) Veröffentlichungstag der Anmeldung:
08.05.91 Patentblatt 91/19

(84) Benannte Vertragsstaaten:
AT BE CH DE ES FR GB GR IT LI LU NL SE

(71) Anmelder: METALPRAECIS BERCHEM +
SCHABERG GESELLSCHAFT FÜR
METALLFORMGEBUNG MBH
Am Dördelmannshof 9-11
W-4650 Gelsenkirchen(DE)

(72) Erfinder: Berchem, Rütger, Dr.

Wallneyer Strasse 29
W-4300 Essen(DE)
Erfinder: Schnitzler, Volkhard, Dipl.-Ing.
Fahrenheitstrasse 34
W-4630 Bochum 5(DE)

(74) Vertreter: Andrejewski, Walter et al
Patentanwälte Dipl.-Phys. Dr. Walter
Andrejewski Dipl.-Ing. Dr.-Ing. Manfred
Honke Dipl.-Phys. Dr. Karl Gerhard Masch
Theaterplatz 3, Postfach 10 02 54
W-4300 Essen 1(DE)

(54) Verfahren zur Herstellung einer Implantat-Gelenkprothese.

(57) Verfahren zur Herstellung einer Implantat-Gelenkprothese, die einen in den Knochenkanal eines Röhrenknochens einzusetzenden Schaft (2) mit Kernbereich und Oberflächenbereich (3) aufweist, wobei die Geometrie des Knochenkanals, z. B. mit einer Durchstrahlungsmethode, ermittelt und nach Maßgabe der ermittelten Geometrie der Schaft (2) dimensioniert wird. Nach Maßgabe der Geometrie des Knochenkanals wird die Geometrieschwerelinie (1) bestimmt. Danach wird der Kernbereich des Schaftes (2) mit Hilfe der Finite Elemente Analysis nach Maßgabe der Geometrie des Knochenkanals festgelegt. Die Randbedingungen a) hinterschneidungsfreie Schaftform und b) Gleichspannungsverteilung bei den von der Implantat-Gelenkprothese aufzunehmenden Regelbeanspruchungen im Übergangsbereich Schaft/Knochen werden beachtet. Nach Maßgabe des Resultates von b) wird auf dem Kernbereich der Oberflächenbereich (3) eingerichtet.

Fig.2

# VERFAHREN ZUR HERSTELLUNG EINER IMPLANTAT-GELENKPROTHESE

Die Erfindung betrifft ein Verfahren zur Herstellung einer Implantat-Gelenkprothese, die einen in den Knochenkanal eines Röhrenknochens einzusetzenden Schaft mit Kernbereich und Oberflächenbereich aufweist, wobei die Geometrie des Knochenkanals, z. B. mit einer Durchstrahlungsmethode, ermittelt und nach Maßgabe der ermittelten Geometrie der Schaft dimensioniert wird. Das Ergebnis ist zumeist eine sogenannte Individualprothese. - Der Kernbereich ist der Bereich, der bei einer solchen Implantat-Gelenkprothese dem Knochenkanal nur verhältnismäßig grob angepaßt ist. Durch Modifizierung des Oberflächenbereiches erfolgt die individuelle Anpassung. Der Oberflächenbereich kann an dem Kernbereich ausgebildet oder angebracht sein.

Im Rahmen der bekannten Maßnahmen, von denen die Erfindung ausgeht (DE-OS 37 40 438) ist der Schaft vorgeformt. Er wird danach durch eine Beschichtung aus Knochenersatzmaterial (z. B. in der Form von hydroxylapatit) nach Maßgabe der Geometrie des Knochenkanals eingerichtet, und zwar derart, daß die Beschichtung aus dem Knochenersatzmaterial in den Belastungsbereichen eine Schichtdicke aufweist, die um ein mehrfaches größer ist als in den Bereichen reduzierter Belastung. Wo die Belastungsbereiche liegen, kann bei einem Patienten, bei dem eine Gelenkprothese implantiert werden soll, ohne zu großen Aufwand ermittelt werden, nämlich anhand von Röntgenbildern und mit den Hilfsmitteln der modernen rechnergestützten technischen Mechanik. Im übrigen leisten insoweit die heute in der Medizin vielfach üblichen Computertomographien eine gute Hilfe. Der vorgeformte Schaft kann ein Serienprodukt sein, dessen Geometrie üblichen Mittelwerten folgt. Aus einer Vielzahl derartiger Schäfte wird ein möglichst gut passender ausge wählt. Der Schaft kann aber auch individuell vorgeformt sein, z. B. nach Maßgabe des Umfanges und der Querschnitte des Knochenkanals, in den der Schaft eingesetzt werden soll. Im Rahmen der bekannten Maßnahmen werden die Beanspruchungen zwar ermittelt, jedoch so hingenommen, wie sie sich einstellen. Es ergeben sich zumeist singuläre Beanspruchungsbereiche. Nach Maßgabe dieser unterschiedlichen Beanspruchungen an dem Schaft werden die Beschichtungen aus dem Knochenersatzmaterial aufgebracht.

Demgegenüber liegt der Erfindung die Aufgabe zugrunde, ein Verfahren zur Herstellung einer Implantat-Gelenkprothese anzugeben, welches bei der Herstellung der Implantat-Gelenkprothese zu besonders gut adaptierten Schäften führt, die nicht nur in geometrischer Hinsicht dem Knochenkanal angepaßt sind, sondern darüber hinaus die Beanspruchungen sehr gleichmäßig aufnehmen.

Zur Lösung dieser Aufgabe lehrt die Erfindung, ausgehend von der eingangs beschriebenen Datenaufnahme, daß zuerst nach Maßgabe der Geometrie des Knochenkanals die Geometrieschwerelinie bestimmt sowie danach der Schaft mit Hilfe der Finite Elemente Analysis nach Maßgabe der Geometrie des Knochenkanals festgelegt wird, und zwar mit den Randbedingungen

a) hinterschneidungsfreie Schaftform,

b) Gleichspannungsverteilung bei den von der Implantat-Gelenkprothese aufzunehmenden Regelbeanspruchungen im Übergangsbereich Schaft/Knochen, und daß unter Beachtung von a) sowie nach Maßgabe des Resultates aus b) auf dem Kernbereich der Oberflächenbereich eingerichtet wird. - Die Geometrieschwerelinie verbindet die Flächenschwerpunkte aller Querschnitte des Knochenkanals längs seiner Achse. Die Freiheit von Hinterschneidungen stellt sicher, daß die erfindungsgemäß hergestellte Implantat-Gelenkprothese stets ohne Schwierigkeiten eingesetzt werden kann.

Eine Implantat-Gelenkprothese ist ein Tragwerk, und zwar in Verbindung mit dem zugeordneten Knochen ein komplexes Tragwerk. Beim Entwurf und bei der Bemessung eines Tragwerkes wird in der technischen Mechanik vor allem die Spannungsverteilung bestimmt. Gelegentlich wird es auch notwendig sein, in bestimmten Punkten des Tragwerkes die Verschiebungen im Sinne der klassischen technischen Mechanik zu berechnen, um abzuschätzen, ob vorgegebene Höchstwerte überschritten werden. Die Spannungsverteilung soll verträglich sein, nämlich der Bedingung des Gleichgewichtes zwischen den inneren und äußeren Kräften genügen. Die Erfindung beruht auf der Erkenntnis, daß es gerade bei Implantat-Gelenkprothesen auf diese Verträglichkeit ankommt. Um für eine gegebene Bemessungsaufgabe ein System von Spannungen und Verformungen zu erhalten, müssen in der Praxis zunächst die Grundgleichungen des Problems aufgestellt werden, die den Gleichgewichts- und Verträglichkeitsbedingungen ausreichend genügen. In der Regel wird es schwierig sein, die Gleichungen den besonderen Gegebenheiten der Geometrie, Belastung und Materialeigenschaften anzupassen, -und auch dieses gilt insbesondere für Implantat-Gelenkprothesen. Bisher hat man gewisse Ungenauigkeiten in Kauf genommen. Demgegenüber nutzt die Erfindung die Entwicklung der modernen elektronischen Rechenanlagen, die die Möglichkeit für solche Berechnungen wesentlich vergrößert und verbessert haben. Es sind damit numerische Lösungen möglich, und zwar mit der Finite Elemente Analysis. Der grundsätzliche Gedanke dieser Methode basiert auf der Modellvorstellung eines Kontinuums als einer Zusammensetzung von Teilbereichen, eben den finiten Elementen. In jedem Teilbereich wird das Verhalten durch einen Satz von Ansatzfunktionen beschrieben, die die Spannungen und Verschiebungen in diesen Teilbereichen wiedergeben. Das ist ein Problem der Software-Entwicklung, die erfindungsgemäß unter den angegebenen besonderen Voraussetzungen oder Randbedingungen erfolgt, nämlich hinterschneidungsfreie Schaftform sowie Gleichspannungsverteilung bei den von der Implantat-Gelenkprothese aufzunehmenden Regelbeanspruchungen im Übergangsbereich Schaft/Knochen. Erfindungsgemäß werden nicht mehr unterschiedliche Beanspruchungsbereiche und damit Spannungsverteilungen zugelassen, die der unterschiedlich dicken Beschichtung mit Knochenersatzmaterial bedürfen. Erfindungsgemäß werden die Voraussetzungen für eine Gleichspannungsverteilung geschaffen, die sich einstellt, wenn der Schaft der Implantat-Gelenkprothese in den Knochenkanal eingeführt wird.

Im einzelnen kann die Optimierung bis zur Gleichspannungsverteilung auf verschiedene Weise durchgeführt werden. Eine Ausführungsform der Erfindung ist dadurch gekennzeichnet, daß der Oberflächenbereich aus vorstehenden Rippen und/oder Noppen aufgebaut wird. Eine andere Ausführungsform ist dadurch gekennzeichnet, daß der Oberflächenbereich als Beschichtung aufgebracht wird, die beispielsweise auch aus einem Knochenersatzmaterial bestehen kann. Überraschenderweise kommt man zu einer sehr homogenen Gleichspannungsverteilung, wenn zunächst die Geometrieschwerelinie bestimmt und beim weiteren Aufbau des Schaftes an diese angeschlossen wird.

Im folgenden wird die Erfindung anhand einer lediglich ein Ausführungsbeispiel darstellenden Zeichnung ausführlicher erläutert. Es zeigen in schematischer Darstellung

Fig. 1 die Geometrieschwerelinie eines Knochenkanals,

Fig. 2 die Ansicht des entsprechenden Schaftes einer Implantat-Gelenkprothese, teilweise im Schnitt,

Fig. 3 den vergrößerten Ausschnitt aus dem Gegenstand der Fig. 2.

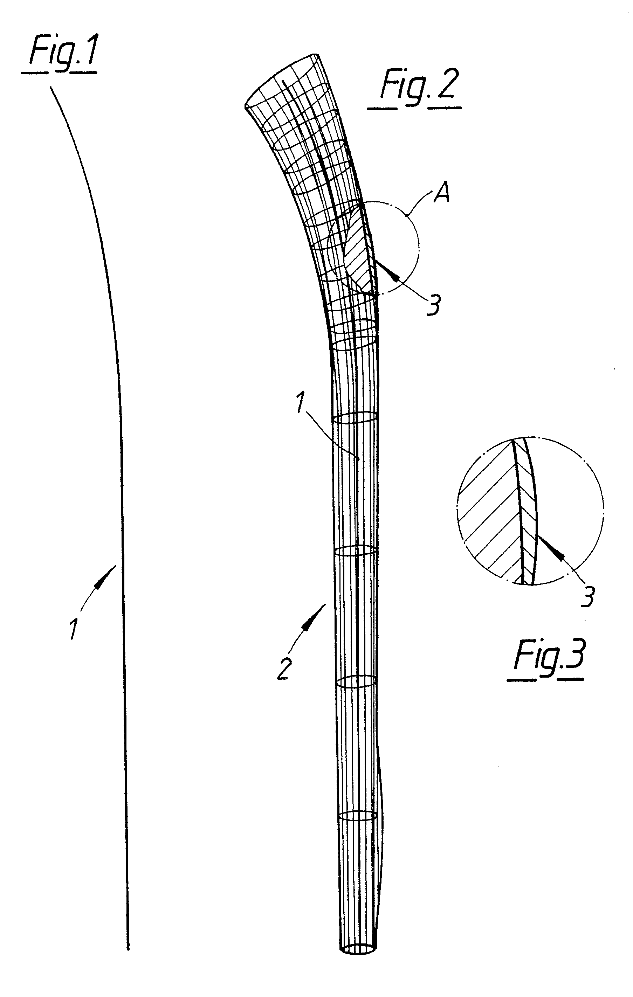

Zur Herstellung einer individuellen Implantat-Gelenkprothese in Form einer Hüftgelenkprothese wurde zunächst die beschriebene Datenaufnahme durchgeführt, d. h. die Geometrie des Knochenkanals in die der Schaft der Gelenkprothese einzusetzen ist, festgelegt und vermessen. Daraus wurde die in Fig. 1 dargestellte Geometrieschwerelinie 1 ermittelt. Sie kann mehrfach, zum Beispiel auch aus der Zeichenebene der Fig. 1 heraus, gekrümmt verlaufen. Es versteht sich, daß die Krümmungen nicht sehr groß sind. Groß ist allerdings ihr Einfluß auf den Sitz und die Haltbarkeit der eingesetzten Implantat-Gelenkprothese.

Fig. 2 zeigt den Schaft 2, der mit Hilfe der Finite Elemente Analysis wie eingangs erläutert festgelegt wurde. Eingezeichnet wurden ringförmige Querkurven, und zwar entsprechend den zugeordneten Schnittflächen des Knochenkanals. Die Geometrieschwerelinie 1 durchdringt die Flächenschwerpunkte dieser Schnittflächen und damit auch der von den Querkurven eingeschlossenen Flächen. Der vergrößerte Ausschnitt A in Fig. 3 zeigt eine Gestaltung des Oberflächenbereiches 3, mit der die nach dem erfindungsgemäßen Verfahren hergestellte Implantat-Gelenkprothese auf Spannungsverteilung optimiert wurde.

Die zur Lehre der Erfindung gehörende Datenaufnahme und die angeschlossenen Berechnungen sind mit den Hilfsmitteln der modernen Computertechnik ohne weiteres automatisch durchzuführen.

Es versteht sich, daß nach dem erfindungsgemäßen Verfahren Implantat-Gelenkprothesen, bis auf die Gleichspannungsoptimierung, auch vorgefertigt werden können, und zwar in verschiedenen Größen und Formen, die statistischen Untersuchungen bei Patienten entsprechen, die mit Implantat-Gelenkprothesen versorgt werden müssen. Diese vorgefertigten Implantat-Gelenkprothesen werden dann vom Arzt durch Auswahl einer gewissen Grobanpassung unterworfen und wie beschrieben weiter optimiert.

## Ansprüche

1. Verfahren zur Herstellung einer Implantat-Gelenkprothese, die einen in den Knochenkanal eines Röhrenknochens einzusetzenden Schaft mit Kernbereich und Oberflächenbereich aufweist, wobei die Geometrie des Knochenkanals, z. B. mit einer Durchstrahlungsmethode, ermittelt und nach Maßgabe der ermittelten Geometrie der Schaft dimensioniert wird, **dadurch gekennzeichnet,** daß zuerst nach Maßgabe der Geometrie des Knochenkanals die Geometrieschwerelinie bestimmt sowie danach der Kernbereich des Schaftes mit Hilfe der Finite Elemente Analysis nach Maßgabe der Geometrie des Knochenkanals festgelegt wird und zwar mit den Randbedingungen

a) hinterschneidungsfreie Schaftform,

b) Gleichspannungsverteilung bei den von der Implantat-Gelenkprothese aufzunehmenden Regelbeanspruchungen im Übergangsbereich Schaft/Knochen,

und daß unter Beachtung von a) und nach Maßgabe des Resultates von b) auf dem Kernbereich der Oberflächenbereich eingerichtet wird.

2. Verfahren nach Anspruch 1, dadurch gekennzeichnet, daß der Oberflächenbereich aus vorstehenden Rippen und/oder Noppen aufgebaut wird.

3. Verfahren nach Anspruch 1, dadurch gekennzeichnet, daß der Oberflächenbereich als Beschichtung aufgebracht wird.

_Fig.1_

_Fig.2_

_Fig.3_

A

3

1

1

2

3

3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | LE NOUVEL AUTOMATISME, Band 30, Nr. 55, Juni 1985, Seiten 50-54, Paris, FR; J.-C. MORETTON et al.: "Problèmes de prothèse, problèmes de CFAO" * Seiten 51-54; Figur 4 * | 1 | A 61 F 2/30 |
| Y | IDEM --- | 2,3 | |
| Y | CH-A- 663 149 (SULZER) * Zusammenfassung; Seite 2, Spalte 2, Zeilen 20-28; Figuren * | 2 | |
| A | --- | 1 | |
| Y,D | DE-A-3 740 438 (METALPRAECIS) * Spalte 2, Zeilen 44-52; Spalte 2, Zeile 64 - Spalte 3, Zeile 6; Figuren * --- | 3 | |
| A | FR-A-2 577 697 (CHALMOND) * Zusammenfassung; Figuren * --- | 1 | |
| A | JOURNAL OF BIOMECHANICS, Band 16, Nr. 9, 1983, Seiten 727-742, Pergamon Press Ltd, GB; A. ROHLMANN et al.: "Finite-element-analysis and experimental investigation in a femur with hip endoprosthesis" * Das ganze Dokument * --- | 1 | RECHERCHIERTE SACHGEBIETE (Int. Cl.5) A 61 F |
| A | EP-A-0 144 574 (WITZEL) * Seite 13, Zeilen 7-9; Figur 5 * --- | 3 | |
| A | ENGINEERING IN MEDICINE, Band 9, Nr. 3, 1980, Seiten 143-146, MEP Ltd; N.L. SVENSSON et al.: "Finite element analysis of a simulated total hip replacement" --- -/- | | |

Der vorliegende Recherchenbericht wurde für alle Patentansprüche erstellt

| Recherchenort | Abschlußdatum der Recherche | Prüfer |
|---|---|---|
| DEN HAAG | 28-06-1990 | KLEIN C. |

EPO FORM 1503 03.82 (P0403)

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | MEDICAL & BIOLOGICAL ENGINEERING & COMPUTING, Band 24, Nr. 3, Mai 1986, Seiten 267-274, IFMBE, London, GB; D. SIU et al.: "Three-dimensional reconstruction of joint surfaces using a microcomputer"<br>--- | | |
| A | EP-A-0 255 797 (MECRON)<br>--- | | |
| A | EP-A-0 038 908 (WALDEMAR LINK)<br>----- | | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| DEN HAAG | 28-06-1990 | KLEIN C. |